# EUROPEAN PATENT APPLICATION

(11) **EP 0 908 161 A1**
(43) Date of publication of application: **14.04.1999**
(21) Application number: 98117200.0
(22) Date of filing: 09.09.1998
(51) Int. Cl.: A61F 11/00, A61M 1/00

(54) **Safety accessory for fabric and wax cones used in cleaning the ear cavity**

(30) Priority: 16.09.1997 IT FO970022
(71) Applicant: Otosan di Gianardi Massimiliano, 47100 Forli (IT)
(72) Inventor: Gianardi, Massimiliano, 47100 Forli (IT)
(74) Representative: Modiano, Guido, Dr.-Ing.

(57) **Abstract**

An accessory (A) constituted by a simple tube (3) on the outside of which a collar (4) is applied which protrudes from the intermediate region of the tube enough to rest against the conical surface of the cone (1) simultaneously with the lower end of the tube (3), thus arranging the accessory (A) coaxially to the cone (1). The outside diameters of the tube (3) and of the collar (4) allow to arrange the accessory below the self-extinguishing collar (2) of the cone (1). The interspace formed by the tube (3) with the cone (1) and closed at the bottom by the collar (4) retains and collects any excess molten wax.

## Description

The present invention relates to a safety accessory for fabric and wax cones currently used to clean the ear cavity, i.e., to remove earwax from the ear without accessing the region where said substance has accumulated with means which might compromise the integrity of said region and most of all of the eardrum.

Said cones, formed by rolling up a strip of cotton or other suitable fabric adequately impregnated with beeswax and/or paraffin, are hollow and open at both ends.

In order to remove earwax from the ear with one of these cones it is sufficient to set fire to its widest end and insert the opposite one in the ear cavity.

During this operation, the head lies laterally on a horizontal resting surface, so that the cone, inserted in the ear and held with one hand, is substantially vertical so that it burns with a gradual and uniform combustion. In the initial step, the fumes and hot air generated by combustion descend to the eardrum barrier and soften the earwax. Then the earwax, the impurities and any water residues are aspirated and collected inside the cone due to a sort of "stack effect".

Understandably, said cones are used only partially and are therefore extinguished after approximately 2/3 of their length has been burned.

In most cases, ear cavity cleaning is performed by a single person; since it is not advisable to interrupt the suction effect of the cone to check the approach of the combustion toward the fingers that hold the cone vertically, said cone is designed to self-extinguish at a preset safety level by having a portion which is covered externally with incombustible material or is impregnated with known fireproof substances. Moreover, especially to reassure the user as to any fall of burnt particles or molten wax, the cone is inserted in a protective disk made of metallized cardboard or other suitable material which is provided with a central hole so as to stop below the self-extinguishing region of the cone.

Said disk obviously protects the region surrounding the cone but does not protect the ear cavity, into which excess molten wax not burned up by combustion, ash and burnt particles might drip, although this is a very unlikely possibility.

The aim of the present invention is to eliminate this possibility by means of a particular accessory to be inserted simply in currently manufactured cones without modifying them and without altering their operation, which indeed by means of the accessory is constant throughout the duration of the combustion and the treatment.

Generally speaking, the accessory according to the present invention is an element which is preferably made of plastics and at low costs, and is shaped and sized so that once inserted into the cone from the widest end it can stop coaxially beyond the self-extinguishing region in order to remain intact throughout the treatment.

The main feature of this accessory is that it forces the fumes to pass through a short duct which is coaxial to the cone and is to a large extent surrounded by a sort of annular interspace closed at the bottom to retain any excess molten wax that might fall thereon from above.

Further characteristics and advantages of the present invention will become apparent from the following detailed description of preferred embodiments thereof, illustrated only by way of non-limitative example in the accompanying drawings, wherein:
Figure 1 is an elevation view of the fabric and wax cone during use;
Figures 2-4; 5-7; 8-10; 11-13 and 14-16 respectively relate to five possible embodiments of the accessory, each of which is shown in a respective drawing which comprises a side view, a top view and a longitudinal sectional view inside the cone of Figure 1.

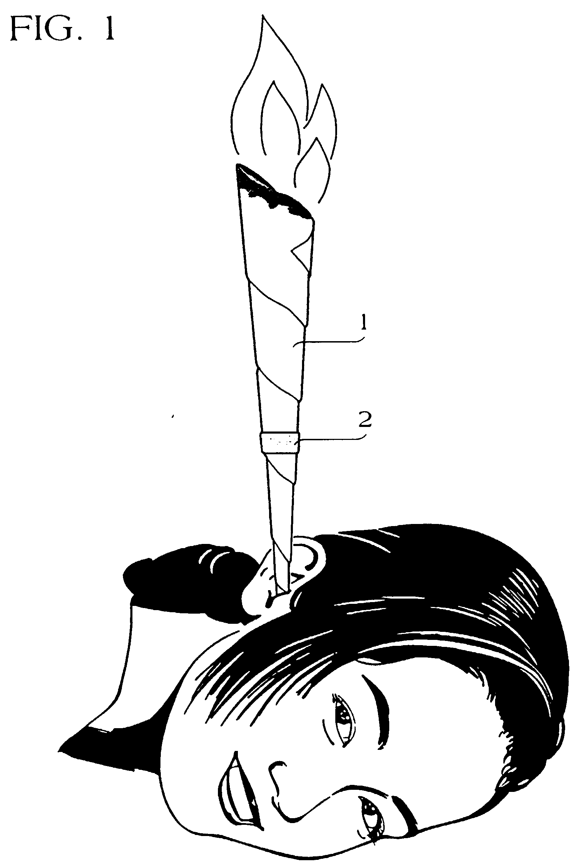

Before describing the five versions or embodiments of the invention, it should specified that the fact that their use has been shown in a cone 1 provided with a self-extinguishing collar 2 (Figure 1) does not exclude the validity of said invention for other cones equipped in other ways for self-extinguishing or even for cones completed by an external protective disk.

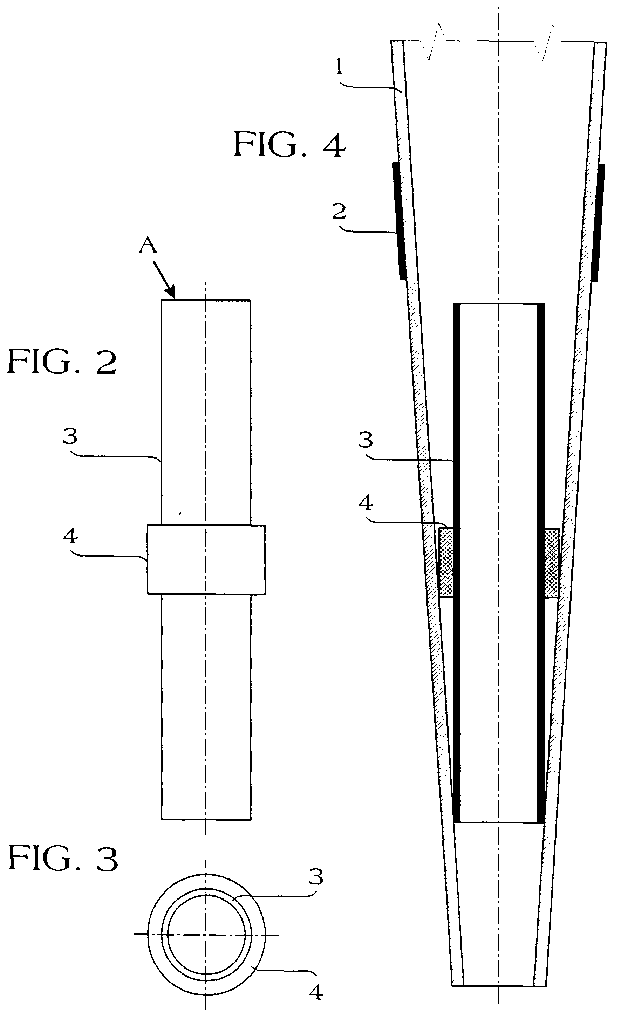

According to the embodiment shown in Figures 2-4, the accessory A is constituted by a simple short tube 3 on the outside whereof a collar 4 is applied; said collar, preferably arranged in the intermediate region, protrudes enough to rest against the conical surface simultaneously with the lower end of the tube 3, thus giving to the accessory an arrangement which is coaxial to the cone.

Furthermore, the collar 4 closes at the bottom the annular interspace which lies between the inner surface of the cone 1 and the outer surface of the tube 3 and is meant to collect any excess molten wax which might otherwise drip into the ear cavity or at least accumulate and obstruct the passage of the fumes.

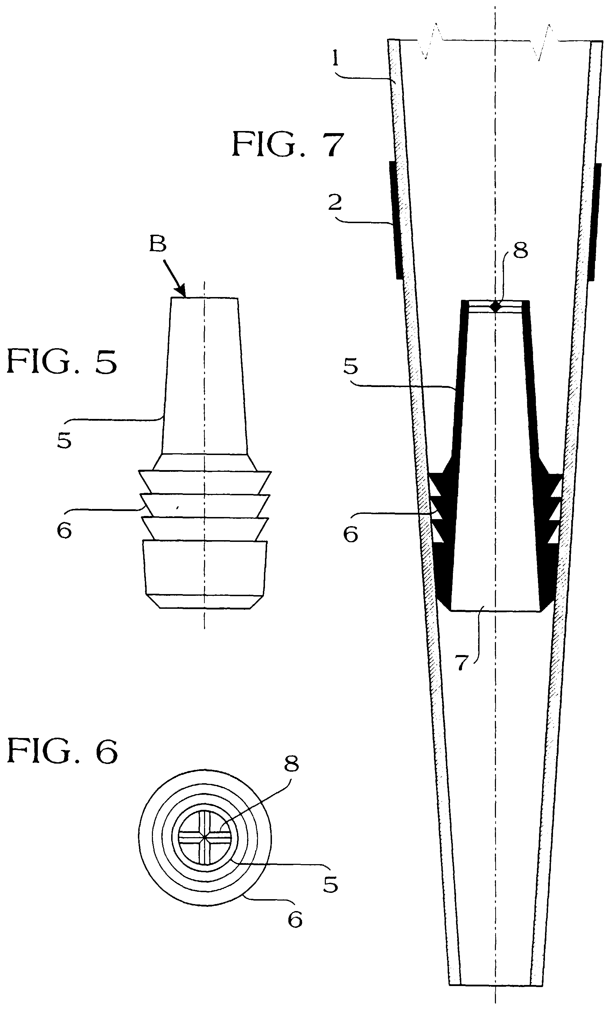

According to the embodiment of Figures 5-7, the accessory B is an element which has a circular shape in plan view, with a frustum-shaped cavity 7 running along the entire length of the element and tapers toward the upper end, where it is possible to provide a protective grille 8 directly during molding.

In its external configuration, the accessory B has a frustum-shaped upper portion 5 which tapers upward, while the lower portion 6, since it must arrange the element coaxially to the cone 1, has a general configuration which tapers downward so as to match the taper of said cone 1, although it is crossed transversely by triangular grooves in order to assume a suitable toothed grip profile.

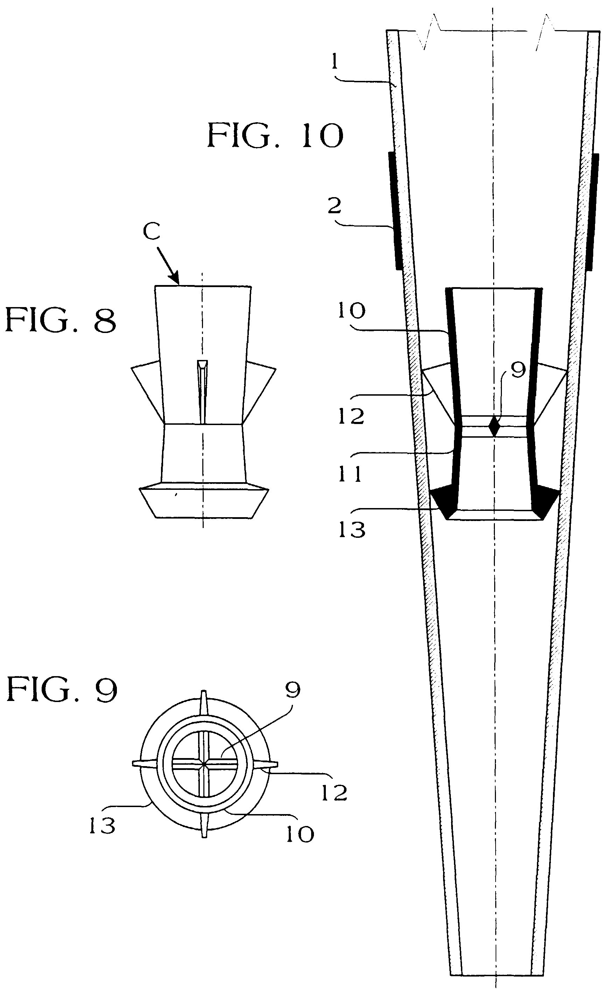

According to the embodiment shown in Figures 8-10, the accessory C is a hollow element which has a circular cross-section and tapers conically from both ends toward an intermediate region, where it is possible to provide a protective grille 9 directly during molding.

Four triangular fins 12 protrude outside the upper frustum-shaped portion 10, while a collar 13 protrudes outward from the edge of the lower frustum-shaped portion 11 and is shaped so as to form a guide for firmer grip on the cone 1 together with the centering fins 12.

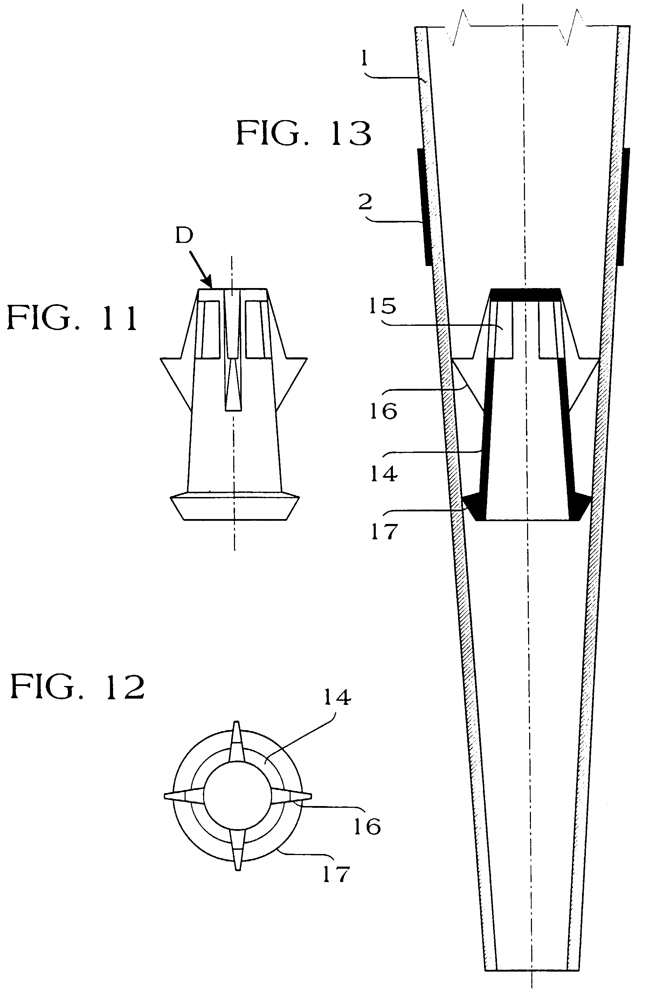

According to the embodiment of Figures 11-13, the accessory D is a predominantly frustum-shaped hollow element which widens toward the lower open end and is closed at the top; four lateral openings 15 for the passage of the fumes are provided in the frustum-shaped wall 14 above the four fins 16 and, together with the collar 17 protruding from the lower edge of the element so as to form a guide, ensure the coaxiality and grip of the accessory D inside the cone 1.

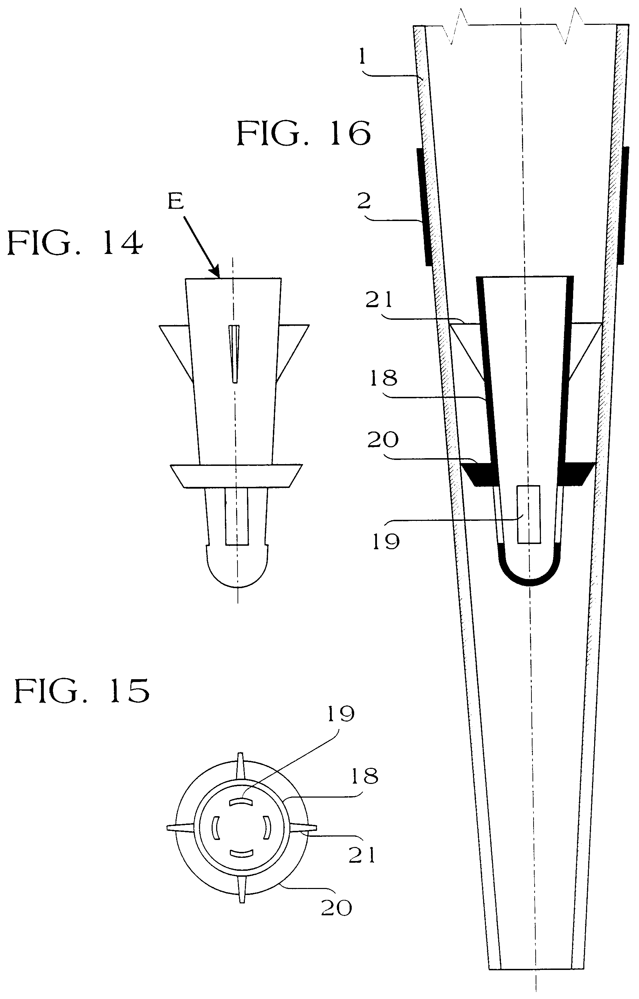

According to the embodiment of Figures 14-16, the accessory E is a hollow and predominantly frustum-shaped element which widens toward the open upper end and is closed at the lower end so as to form a hemispherical dome; four lateral openings 19 for the passage of the fumes are formed in the vicinity in the frustum-shaped wall 18 below the collar 20, which is shaped so as to form a guide in order to ensure, together with the overlying four fins 21, the coaxiality and the grip of the accessory E in the cone 1.

It is evident from the above description and illustration that all the described possible embodiments allow, according to the aim and objects of the present invention, to retain any excess molten wax in a sort of annular interspace which is closed at the bottom and surrounds the fume passage duct.

Moreover, in order to retain any burnt particles that might reach the fume passage duct, some of the accessory embodiments (B and C) can be completed with a protective grille, while the others (D and E) already have lateral openings because the fume duct is closed at one of its two ends.

Obviously, without altering the general characteristics that have been described and illustrated, the above embodiments are susceptible of modifications and variations which are nonetheless within the claim scope of the present invention.

The disclosures in Italian Patent Application No. FO97A000022 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A safety accessory for fabric and wax cones used to clean the ear cavity, consisting of an element made of plastics or other suitable material which is meant to prevent the descent of any drops of excess molten wax, ash and unburned particles into the ear cavity, characterized in that it is shaped and sized so that when it is inserted from the widest end of the fabric and wax cone (1) the element (A,B,C,D,E) can stop coaxially thereto, in the region that is meant to remain unburned, for forcing the fumes to pass through a short duct (3) which is coaxial to the cone (1) and is to a large extent surrounded by a sort of annular interspace which is closed at the bottom to retain any excess molten wax which might fall from above.

2. The safety accessory according to claim 1, characterized in that said element (A) is constituted by a simple tube (3) on the outside of which a collar (4) is applied, said collar being preferably arranged in the intermediate region and protruding enough to rest against the conical surface simultaneously with the lower end of the tube (3), thus arranging the accessory coaxially to the cone.

3. The safety accessory according to claim 1, characterized in that said element (B) is an element which has a circular shape in plan view and contains a frustum-shaped cavity (7) which runs along the entire length of the element and tapers toward the upper end, where it is possible to provide a protective grille (8), said element having, on the outside, an upper frustum-shaped portion (5) which tapers upward and a lower portion (6) which, since it must arrange the element coaxially to the cone (1), is generally shaped so as to taper downward to match the taper of said cone (1), and is crossed transversely by triangular grooves such as to assume a suitable toothed grip profile.

4. The safety accessory according to claim 1, characterized in that said element (C) is a hollow element which has a circular cross-section and tapers conically from its two ends toward an intermediate region where it is possible to provide a protective grille (9), said element having four triangular fins (12) protruding outside the upper frustum-shaped portion (10) and a collar (13) which protrudes outward from the edge of the lower frustum-shaped portion (11) and forms a guide for safer grip to the cone (1) together with said centering fins (12).

5. The safety accessory according to claim 1, characterized in that said element (D) is a hollow element which has a predominantly frustum-shaped configuration, widens toward the open lower end and is closed at the top, lateral openings (15) adapted for the passage of the fumes being provided in the frustum-shaped wall (14) above four fins (16) which, together with the collar (17), protruding so as to form a guide from the lower edge of the element, ensure the coaxiality and grip of the element (D) in the cone (1).

6. The safety accessory according to claim 1, characterized in that said element (E) is a hollow and predominantly frustum-shaped element which widens toward its upper open end and is closed at its lower end, which lower end is shaped like a hemispherical dome, lateral openings (19) adapted for the passage of the fumes being further provided in the vicinity of a frustum-shaped wall (18) below a collar (20) of the element (E), which is shaped like a guide so as to ensure, together with the four fins (21) overlying the collar, the coaxiality and grip of the element (E) in the cone (1).
